# EUROPEAN PATENT SPECIFICATION

(11) **EP 1 545 376 B1**
(45) Date of publication and mention of the grant of the patent: **15.07.2009**
(21) Application number: 03798615.5
(22) Date of filing: 01.09.2003
(51) Int. Cl.: A61B 19/08

(54) **FLUID COLLECTION POUCH MADE OF FLEXIBLE PLASTIC MATERIAL FOR SURGICAL DRAPES OR TOWELS**
FLÜSSIGKEITSAUFFANGBEUTEL AUS FLEXIBLEM PLASTIKMATERIAL FÜR CHIRURGISCHE ABDECKTÜCHER ODER HANDTÜCHER
SACHET DE COLLECTE DE FLUIDE EN MATIERE PLASTIQUE SOUPLE, DESTINE A DES DRAPS OU DES SERVIETTES CHIRURGICAUX

(30) Priority: 30.09.2002 SE 0202870
(43) Date of publication of application: 29.06.2005
(73) Proprietor: Mölnlycke Health Care AB, 402 52 Göteborg (SE)
(72) Inventor: ANDRESEN, Angelica Brigitta, S-423 41 Torslanda (SE); LAGER, Katarina, S-412 64 Göteborg (SE)
(74) Representative: Hyltner, Jan-Olof
(86) International application number: PCT/SE2003/001351
(87) International publication number: WO 2004/028388

(56) References cited:
- US-A- 4 890 628
- US-A- 6 032 670

## Description

### TECHNICAL FIELD

The present invention relates to a fluid collection pouch made of flexible plastic material for surgical drapes or towels, consisting of two layers and comprising an opening along which said layers are unattached to each other, and two side edges extending on opposite sides of the opening along which said layers are attached to each other. The invention relates also to a method of manufacturing such a pouch.

### BACKGROUND OF THE INVENTION

In surgical interventions the patient is often covered by surgical drapes or towels in order to prevent contamination of surgical staff and operating theatre. If there is a great risk that large amount of fluid will be emitted from the operating site, said fluid can not be absorbed by the drape or the towels but must be taken care of in some other way. An often used solution is to provide fluid collection pouches in the vicinity of the operating site. Such pouches can either be attached in advance to the surgical drape or towel or be separate and applied to the drape or towel in connection with the draping procedure before a surgical intervention. Before a surgical intervention the pouches are packed in a planar state, the layers of the pouch then abutting each other. It is therefore necessary to open the pouch during the draping procedure before operation by removing the abutting layers from each other in such a way that they remain distanced from each other. One way of accomplish this is to provide one or both of the abutting layer of the packed pouch with deformable metallic threads or bands, being deformed during the draping procedure before operation so that said layers be distanced from each other. The presence of such deformable elements makes the pouch more costly and complicates the manufacture thereof. Furthermore, the fluid from the operating site must be guided so as to not flow out over the surgical drape or surgical towels but to the pouch or pouches applied to the drape or towels. One way of accomplishing this is to let the pouch or the pouches cover the whole periphery of the operating site, see for example US 6,032,670. A drawback of such an arrangement is that the collection part of the pouches will be located close to the operating site and may be in the way for the operating staff.

An objective of the present invention is to provide a fluid collection pouch of the type mentioned in the introduction which is easy to manufacture and eliminates or at least significantly reduces the need of deformable threads or bands and furthermore allows a good control of the flow of fluid from the operating site, so that the collection part of the pouch can be located at an optional distance from the operating site.

### SUMMARY OF THE INVENTION

This objective is according to the invention accomplished by a fluid collection pouch made of flexible plastic material for surgical drapes or towels, which consists of two layers and comprises an opening along which said layers are unattached to each other, and two side edges extending on opposite sides of the opening along which said layers are attached to each other, **characterised in that** at least one portion of at least one of the layers of the pouch comprises at least one bellows-fold. By this fold, the pouch is given an increased extension in the height direction, i.e. in a direction perpendicular to the two layers constituting the pouch. The increased extension in the height direction arises partly from the increased amount of material, provided by the fold in said portion, partly by the fact that the material in the fold strive to resume its unfolded form.

In a preferred embodiment, each of the side edge parts of the pouch comprises at least one portion containing a bellows-fold.

In a preferred alternative of this embodiment, one of the two layers constituting the pouch extends beyond the edge of the second layer and has bellows-folded side edges. Preferably, the pouch is manufactured in one piece of a rectangular piece of material, whereby one of the layers of the pouch is constituted by two similarly shaped end parts having the form of right-angled triangles, which are folded in against each other and affixed to each other in a flange-like seam and which together with the end-part of the piece of material over which said two similarly shaped end-parts have been folded constitute the collection part of the pouch having the shape of a cone. Advantageously, drainage pipes or tubes are affixed to the bottom of the cone-shaped collection part.

The folded parts in the bellows-fold are joined to each other in one or several portions along the length of the bellows-folds.

The invention relates also to a method of manufacturing a fluid collection pouch made of flexible plastic material for surgical drapes or towels, consisting of two layers and comprising an opening along which said layer are unattached to each other, and two side edges extending on opposite sides of the opening along which said layers are attached to each other, characterised by the following steps;
providing a pouch blank comprising two identical rectangular layers of flexible plastic material having two opposing long sides and two opposing short sides, said layers being placed on top of each other so that their long and short sides lie edge-to-edge and having the edge parts along one long side and at least one short side joined to each other,
thereafter folding out the long sides from each other until they are located in the same plane, whereby the end or ends of the material piece having the edges of the short sides joined to each other assume a cone-shape by being folded about folding lines inclined to the length direction and by the short side edges joined to each other being folded in against the material piece and extending along the length symmetry axis (A-A) of the pouch,
bellows-folding the longitudinal edge parts of the pouch blank either before or after said folding out of the long sides so that the bellows-folds are located on the same side of the pouch blank as the edges of the short sides that have been folded in, whereby a fluid collection pouch is formed.

In a preferred embodiment, the pouch blank is provided by folding a piece of flexible plastic material having a rectangular shape comprising two opposite long sides and two opposite short sides about a central longitudinal folding line (A-A) so that the long and short sides are located edge-to-edge to each other, whereafter the two end parts folded against each other of at least one of the short sides are joined to each other. After bellows-folding the longitudinal edge parts of the material piece, the transverse edge part of the cone-shaped part or the transverse edge parts of the cone-shaped parts of the formed collection pouch are folded in over or under the edge part in question.

A coupling to drainage pipes or tubes can advantageously be placed between the edge parts of the shorts sides being folded in against each other before these edge parts are folded in against each other and the coupling is joined to these edge parts in connection with the joining thereof.

### BRIEF DESCRIPTION OF THE DRAWINGS

The invention shall now be described with reference to the enclosed Figures, of which;
Figs. 1-7 schematically disclose the different steps for the manufacture of a fluid collection pouch according to an embodiment of the invention,
Fig. 8 discloses a surgical drape provide with a fluid collection pouch manufactured by the method according to Figures 1-7,
Figs. 9-12 show schematic alternative embodiments to provide a pouch blank according to Figure 2, and
Figs. 13 and 14 disclose further embodiments of fluid collection pouches according to the invention.

### DESCRIPTION OF EMBODIMENTS

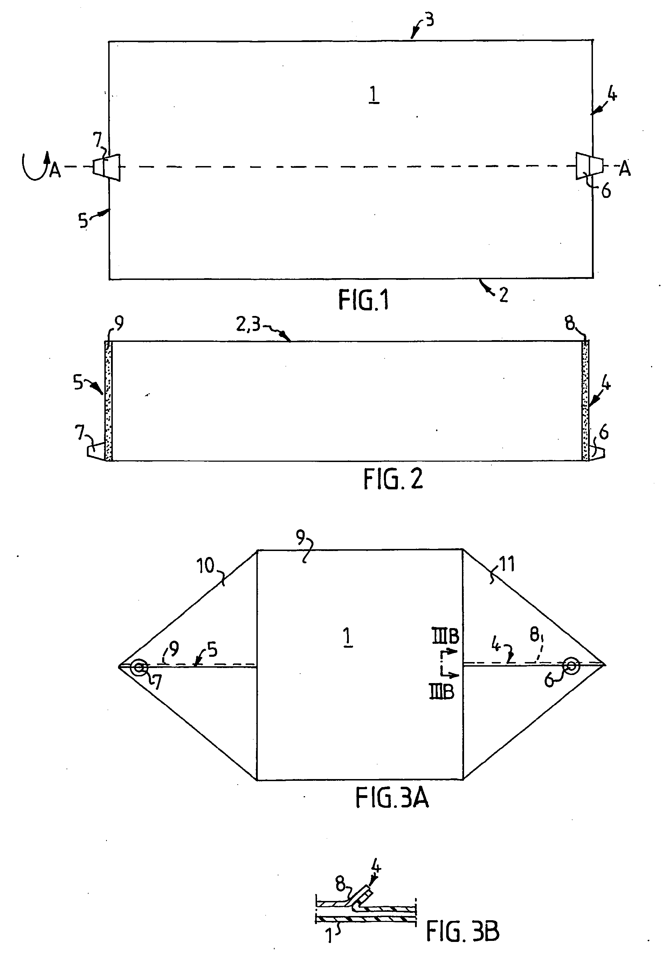

Figures 1-6 disclose schematically the different steps to manufacture a fluid collection pouch according to a preferred embodiment of the invention starting with a rectangular sheet 1 of flexible plastic material. The sheet 1 has two mutually opposite long sides 2,3 and two mutually opposite short sides 4,5. The side of the sheet 1 turned against the viewer of Figure 1 is the upper side of the sheet.

As a first step in the manufacture of a fluid collection pouch, a coupling 6,7 for drainage pipes or tubes is placed in the middle of each short side 4,5 with their tube axes directed along the longitudinal symmetry axis A-A of sheet 1. Thereafter the sheet is folded about the longitudinal symmetry axis A-A so that the long sides 2,3 will lie edge-to-edge. In Figure 1 this folding is symbolised by an arrow.

In Figure 2, the sheet 1 is shown after folding along the longitudinal symmetry axis A-A. In a second step of manufacture the parts folded against each other of the short side edges 4 and 5, respectively are joined to each other by heat welding or gluing, whereby weld or glue seams 8,9 arise. Said seams 8,9 also join the couplings 6,7 to surrounding portions of the side edge parts of the sheet 1.

The couplings 6,7 can of course be placed in other locations along the edge parts of the short sides than the locations shown in Figure 1.

The sheet 1 is then folded back by removing the long side parts from each other. Since the short side parts are joined to each other they can not be removed from each other so the folding back of the sheet 1 from the folded state shown in Figure 2 has the consequence of making the joined short side parts swing in against the upper side of the sheet 1 during simultaneous folding out of the portions of the sheet lying outside of the seams 8,9. After being totally folded back, the sheet has the configuration shown in Figure 3A with a central rectangular portion 9 and two opposite end parts 10,11 having the form of isosceles triangles. By the folding back of the sheet 1, a central one-layered part 9 and two two-layered cone-shaped end parts 10,11 are thus produced. The two-layered cone-shaped end parts 10,11 constitutes collection parts with side edges 10A, 10B and 11A,11B, respectively, said parts being formed from the same plastic sheet. In Figure 3B a portion of the sheet is shown in a sectional view along line IIIB-IIIB in Figure 3A. As is evident from this view, the joining of the portions of short side parts 4 and 5, respectively lying against each other leads to the forming of an erect flange by the above described folding back of the sheet from the state shown in Figure 2.

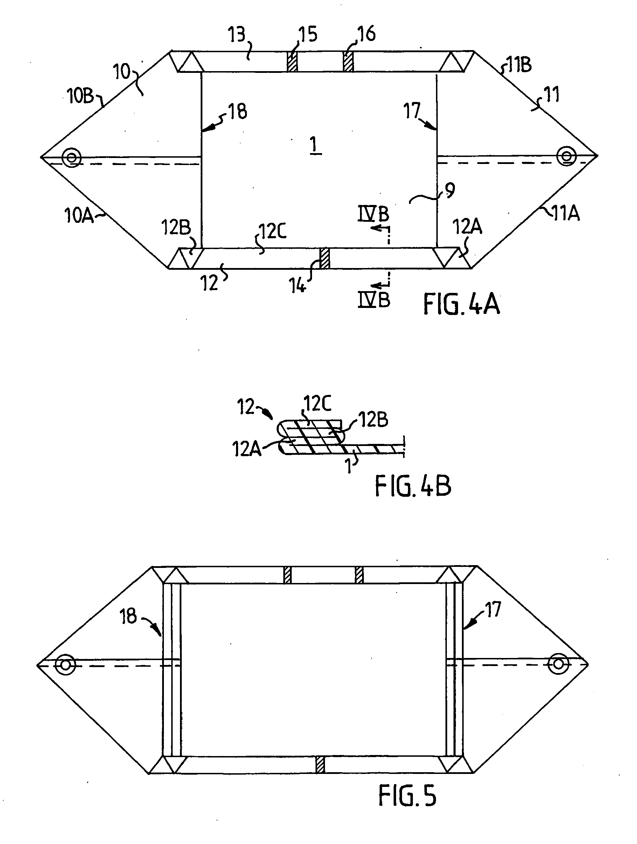

In the next step of manufacture, the long side parts of the folded back sheet 1 are bellows-folded in the way shown in Figures 4A and 4B so that two longitudinal bellows-folds 12,13 are formed. In the central part 9 each such fold will consist of three layers folded onto each other, each such fold being folded in over the respective long side of the sheet 1, as is shown in figure 4B, in which the folded portions 12A, 12B, 12C are folded in over the upper side of the sheet 1. In order to prevent unfolding of the bellows-fold 12,13 due to deformation resistance in the folded plastic material, the bellows-folds are secured by joining together the three layers, that are folded onto each other, and the upper side of sheet 1, over which these layers are folded, at at least one location along the length of these folds. The join can consist of one or several weld points, a weld line or of glue joins. In the disclosed embodiment, the bellows-fold 12 is secured by a weld or glue join 14 while the bellows-fold 13 is secured by two weld or glue joins 15,16 distanced from each other. The joins 14,15,16 should also be distanced from the transverse edges 17,18 of the upper layer in the cone-shaped end parts 10,11 of the folded sheet 1.

By the term bellows-fold is meant folds similar to the folds in a bellows, which are folded such as the fold lines alternatively run along opposite sides of the bellows-fold. The bellows-fold 12 shown in Figure 4B contains two bellows-foldings 12A,12B and 12B,12C, respectively, whereby the portion 12B is common to both of the bellows-foldings. A bellows-fold according to the present application contains at least one such bellows-folding.

Thereafter, the transverse edges 17,18 of the upper layer in the cone-shaped end parts 10,11 of the folded sheet 1 are folded in under the under side of said upper layers and the folds formed are secured by being attached to the upper layers in a suitable way, for example with the aid of double-adhesive tape, glue beads or glue points. The configuration of the sheet 1 after the folding in of the edges 17,18 has been made is shown in Figure 5.

It is of course also possible to fold the transverse edges 17,18 in over the upper side of the upper layers of the cone-shaped end parts 10,11. By such folding of the edges 17,18 an up-lifting of the bellows-fold 12,13 occurs in the region of the transverse edge portions of the cone-shaped end parts 10,11. In order to allow such up-lifting the joins 14-16 securing the bellows-fold must be distanced from the edges 17,18. In order to facilitate the folding in of the transverse edges, the erect flanges constituted by the seams 8,9 are suitably folded against the upper side of the upper layer of the cone-shaped end parts 10,11. This folding of the seams can possibly be done already at the swinging in of the joined short sides against the central part of the sheet 1.

Then the sheet 1 is provided with beads 19,20,21 of adhesive coating on its underside for allowing the sheet 1 to be attached to a surgical drape or towel. In the embodiment shown the beads 19-21 forms a right-angled U. The adhesive coating is protected by a release layer which is removed when the fluid collection pouch formed by the sheet 1 is to be applied to a surgical drape or towel. The adhesive coating can consist of one side of a double-adhesive tape or of beads of glue, e.g. hot melt adhesive.

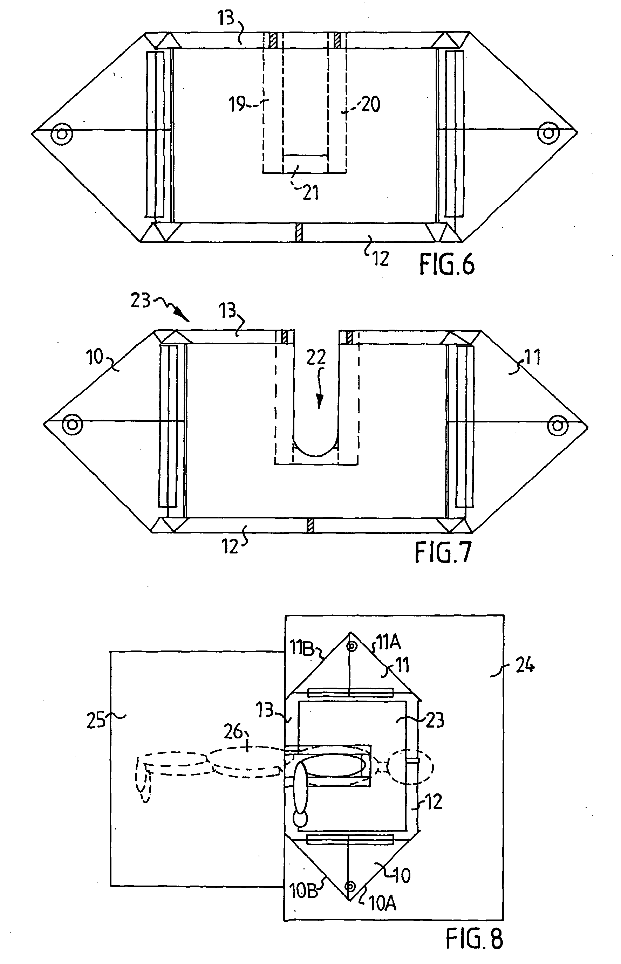

Finally, the material between the beads 19-21 is cut out from the sheet 1 in a way that is evident from Figure 7, disclosing a finalised fluid collection pouch 23 made from the sheet 1 and having two cone-shaped end parts 10,11 for storage or intermediary storage of fluid.

In Figure 8 the fluid collection pouch 23 is shown attached to a surgical towel 24 designed for shoulder operations. In the Figure is also schematically shown a patient draped for a shoulder operation by the surgical towel 24 and a further surgical towel 25. The patient lies on his side and his right arm is passed through an opening in the surgical towel 24 and through the U-formed opening 22 in the fluid collection pouch 23. The portions of the surgical towels 24,25 and fluid collection pouch 23 lying outside of the patient are located below the upwardly turned side of the patient so that fluid emitted from the operating site will flow in a direction towards the cone-shaped end parts 10,11 or towards the bellows-fold 12,13 on the fluid collection pouch 23. The bellows-fold 12,13 forms upstanding barriers, which prevent fluid from running off the central part of the fluid collection pouch 23 and guide the fluid towards the cone-shaped end parts, in which emitted fluid is stored. It is pointed out that as a consequence of the deformation resistance of the material in the bellows-fold, said folds strive to unfold, which lead to that the barriers formed by the bellows-fold are given a greater height than the total sum of the thickness of the three layers being parts of the bellows-folds extending above the bottom layer of the fluid collection pouch in all portion in which the bellows-folds are not secured by a weld or glue join.

The deformation resistance of the pouch material lead also to that the sides of the cone-shaped end parts strive to move away from each other, a tendency that is supported by the above mentioned folding down of the erect flange on the upper sides of the cone-shaped end parts before the folding over of the edge portions 17,18. This folding down will also lead to than one half of the upper layer of the cone-shaped end parts will be located on a slightly higher level than the other half, as is schematically shown in Figure 3B. The overfolded edge portions 17,18 constitutes also a small barrier which to some extent prevent fluid from flowing over the edge of the opening of the cone-shaped end parts. It is pointed out that normally it is enough that the two layers forming the cone-shaped end parts, are spaced a small distance from each other for the described fluid collection pouch to function well. As soon as fluid begins to flow into the cone-shaped end parts, the fluid will move the walls of the cone-shaped end parts away from each other, starting from the bottoms thereof. It is, however, of course possible to provide the openings of the cone-shaped end parts with deformable metal bands, if desired, suitably in connection with the folding over of the edge portions 17,18 of the cone-shaped end parts.

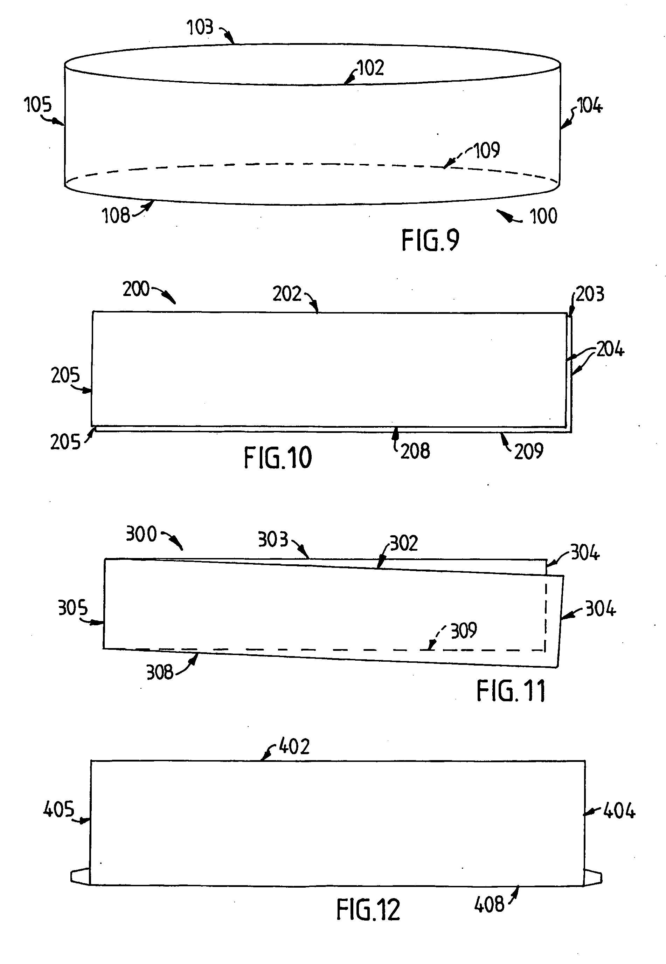

In Figures 9-12 alternative embodiments for the making of a pouch blank according to the invention are shown.

In Figure 9, the pouch blank is formed from an annular piece of plastic 100, which is folded to obtain a rectangular shape with long sides 102,103 and short sides 104,105. In figure 9, is, for the sake of clarity, the piece 100 shown immediately before the folding is finished. To accomplish a pouch blank corresponding to the blank shown in Figure 2, the long side parts 108 and 109 are joined to each other.

In Figure 10, the pouch blank is formed of two identical rectangular pieces of plastic material having two opposite long sides 202, 208 and 203, 209, respectively and two opposite short sides 204 and 205. These rectangular pieces are placed on each other with the long sides and the short sides located edge-to-edge. To accomplish a pouch blank corresponding to the blank shown in Figure 2, the edge parts of the adjacent short sides 204 and 205 of both pieces and long side parts 208 and 209 of both pieces are joined to each other.

In Figure 11, the pouch blank is formed of an elongated rectangular piece of plastic material 300, which is folded about an axis parallel to the short sides 304 so that two identical pieces are formed with two opposite long sides 302,308 and 303, 309, respectively and two opposite short sides 304 and 305. For clarity, the piece 300 is shown immediately before the folding is finished.. To accomplish a pouch blank corresponding to the blank shown in Figure 2, the long side parts 308 and 309 and the short side parts 304 are joined to each other.

In Figure 12 is shown a pouch blank with lower long side edge portions 408 joined to each other by a weld or glue join, and short side edge portions 404,405, which are joined to each other either by a join or by the pouch blank being manufactured from a single piece of plastic material.

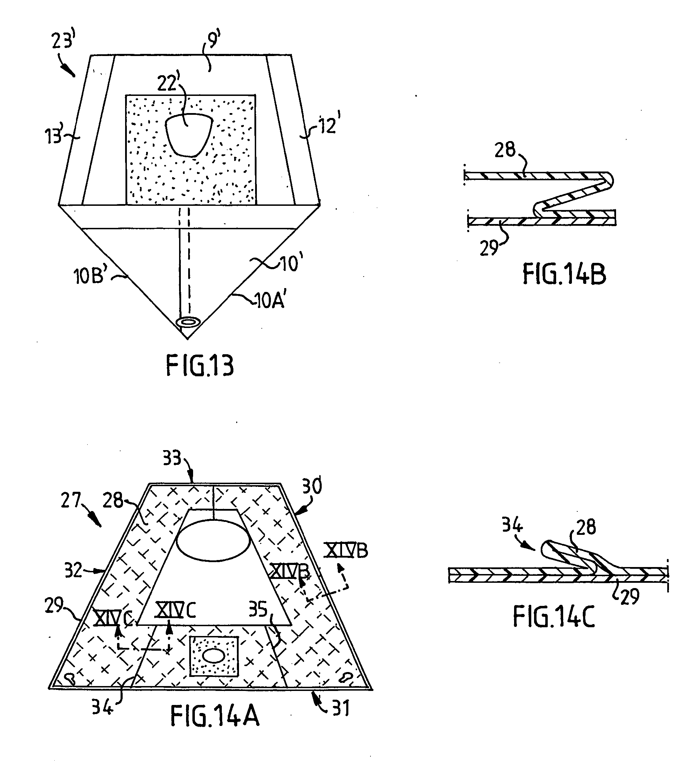

In Figure 13 is shown a fluid collection pouch according to a second embodiment of the invention. This pouch has a great similarity to the pouch shown in Figure 7 and components of this pouch being similar to corresponding components of the pouch according to Figure 7 are given the same reference numerals with the addition of a prime sign. The pouch 23' has only one cone-formed end part 10' and bellows-folds 12', 13', which extend converging to each other in a direction from the cone 10'. Furthermore, the opening 22' is situated in the middle of the one-layer part 9' of the pouch. As for the rest, the pouch 23' in Figure 9 corresponds to the pouch 23 in Figure 7.

In Figures 14A,B,C, a fluid collection pouch according to a third embodiment of the invention is shown. This pouch is made of two separate sheets of which the upper layer 28 is bellows-folded along at least two opposite edges 30,32. In Figure 14B one such bellows-fold is schematically shown. It is of course possible to bellow-fold the upper layer 28 along all its outer edges, i.e. also along the upper and lower edges 31,33 as seen in the figure. Also in this pouch the deformation resistance of the material will distance the two layers 28,29 from each other.

Folds can also be disposed in a direction transverse to the edges of the formed pouches. In such cases, an edge weld is suitably used to secure such folds. In Figure 14A and 14C examples are shown of folds extending in a direction transverse to the edge 31 and are secured by edge welding. It is of course possible to dispose folds anywhere on the formed pouches but for reasons of manufacture it is preferred to make use of the edge joins to secure the folds.

The plastic material used for pouches according to the invention consists suitably of polyethene (PE) but also other plastic materials can be used. The plastic material has a thickness of 30-200 µm.

The embodiments described can of course be modified within the scope of invention. The pouch in Figure 7 can, for example, instead of the U-shaped opening 22 be provided with an opening in the middle. The pouch according to Figure 7 can also be provided with two openings on either side of its transverse symmetry line and then be divided into two pouches by a cut along its transverse symmetry line. At the manufacture of the pouch according to Figure 7, the bellows-folds along the longitudinal sides 2,3 can be performed before the first folding of the sheet 1 along the longitudinal symmetry line A-A instead of after the out-folding made after the first folding. Such a measure will lead to an increased thickness of the upstanding flange joining together the upper sides of the cones and to an increased tendency for the sides of the cones to distance themselves from each other. Furthermore, the number of folds in the bellows-folds can be varied. It is also possible to provide bellows-folds that are situated inside of the edge portions of the long- or short sides and that extend between opposite short or long sides. The pouches can also have other shapes than disclosed in the Figures. The invention shall therefore only be restricted by the content of the appended patent claims.

## Claims

1. A fluid collection pouch (23;23';27) made of flexible plastic material for surgical drapes or towels (24,25), which consists of two layers and comprises an opening along which said layers are unattached to each other, and two side edges (10A,10B,11A,11B; 10A',10B';31,33 AND 30,32) extending on opposite sides of the opening along which said layers are attached to each other, **characterised in that** at least one portion (12,13,10,11;12',13',10'; 30,32) of at least one of the layers of the pouch comprises at least one bellows-fold.

2. The pouch according to Claim 1, **characterised in that** each of the side edge parts of the pouch (12,13; 12',13'; 30,32) comprises at least one portion containing a bellows-fold.

3. The pouch according to Claims 1 or 2, **characterised in that** one (9;9') of the two layers (9,10,11; 9',10') constituting the pouch extends beyond the edge of the second layer and has bellows-folded side edges (12,13;12',13').

4. The pouch according to Claim 3, **characterised in that** the pouch is manufactured of one piece of material.

5. The pouch according to Claim 4, **characterised in that** the pouch (23) is manufactured of a rectangular piece of material (1), whereby one of the layers of the pouch is constituted by two similarly shaped end parts having the form of right-angled triangles, which are folded in against each other and affixed to each other in a flange-like seam (4,5) and which together with the end-part of the piece of material over which said two similarly shaped end-parts have been folded, constitute the collection part (10,11) of the pouch having the shape of a cone.

6. The pouch according to Claim 5, **characterised in that** drainage pipes or tubes are affixed to the bottom of the cone-shaped collection part (10,11).

7. The pouch according to any one of Claims 1-6, **characterised in that** the folded parts in the bellows-folds (12,13) are joined to each other in one or several portions (14,15,16) along the length of the bellows-folds.

8. A method of manufacturing a fluid collection pouch made of flexible plastic material for surgical drapes or towels, consisting of two layers and comprising an opening along which said layer are unattached to each other, and two side edges extending on opposite sides of the opening along which said layers are attached to each other, **characterised by** the following steps;
providing a pouch blank comprising two identical rectangular layers of flexible plastic material having two opposing long sides and two opposing short sides, said layers being placed on top of each other so that their long and short sides lie edge-to-edge and having the edge parts along one long side and at least one short side joined to each other,
thereafter folding out the long sides from each other until they are located in the same plane, whereby the end or ends (10,11) of the material piece having the edges of the short sides joined to each other assume a cone-shape by being folded about folding lines inclined to the length direction and by the short side edges joined to each other being folded in against the material piece and extending along the length symmetry axis (A-A) of the pouch,
bellows-folding the longitudinal edge parts of the pouch blank either before or after said folding out of the long sides so that the bellows-folds (12,13) are located on the same side of the pouch blank (1) as the edges of the short sides that have been folded in, whereby a fluid collection pouch is formed.

9. The method according to Claim 8, **characterised by** providing the pouch blank by folding a piece of flexible plastic material (1) having a rectangular shape comprising two opposite long sides (2,3) and two opposite short sides about a central longitudinal folding line (A-A) so that the long and short sides are located edge-to-edge to each other, whereafter the two end parts folded against each other of at least one of the short sides are joined to each other.

10. The method according to Claim 8 or 9, **characterised by** folding the transverse edge part of the cone-shaped part or the transverse edge parts (17,18) of the cone-shaped parts (10,11) of the formed collection pouch in over or under the edge part in question after the bellows-folding of the longitudinal edge parts of the material piece.

11. The method according to Claim 8, 9 or 10, **characterised by** placing a coupling (6,7) to drainage pipes or tubes between the edge parts of the shorts sides (4,5) being folded in against each other.

## Patentansprüche

1. Fluidsammelbeutel (23; 23'; 27) aus einem flexiblen Kunststoffmaterial für chirurgische Vorhänge oder Handtücher (24, 25), der aus zwei Schichten besteht und eine Öffnung, entlang der die Schichten nicht aneinander angebracht sind, und zwei Seitenkanten (10A, 10B, 11A, 11B; 10A', 10B'; 31, 33 und 30, 32) aufweist, die sich auf gegenüberliegenden Seiten der Öffnung erstrecken, entlang derer die Schichten aneinander angebracht sind, **dadurch gekennzeichnet, dass** zumindest ein Abschnitt (12, 13, 10, 11; 12', 13', 10'; 30, 32) von zumindest einer der Schichten des Beutels zumindest eine Ausgleichsfalte aufweist.

2. Beutel nach Anspruch 1, **dadurch gekennzeichnet, dass** jeder der Seitenkantenteile des Beutels (12, 13; 12', 13'; 30, 32) zumindest einen Abschnitt aufweist, der eine Ausgleichsfalte enthält.

3. Beutel nach Anspruch 1 oder 2, **dadurch gekennzeichnet, dass** sich eine (9; 9') der zwei Schichten (9, 10, 11; 9', 10'), die den Beutel bilden, über die Kante der zweiten Schicht hinaus erstreckt und mit Ausgleichsfalten versehene Seitenkanten (12, 13; 12', 13') aufweist,

4. Beutel nach Anspruch 3, **dadurch gekennzeichnet, dass** der Beutel aus einem Materialstück hergestellt ist.

5. Beutel nach Anspruch 4, **dadurch gekennzeichnet, dass** der Beutel (23) aus einem rechteckigen Materialstück (1) hergestellt ist, wodurch eine der Schichten des Beutels durch zwei ähnlich geformte Endteile mit der Gestalt rechtwinkliger Dreiecke gebildet ist, die gegeneinander eingefaltet und aneinander in einer bundähnlichen Naht (4, 5) befestigt sind und die zusammen mit dem Endteil des Materialstücks, über dem die zwei ähnlich geformten Endteile gefaltet worden sind, den Sammelteil (10, 11) des Beutels bilden, der die Form eines Kegels hat.

6. Beutel nach Anspruch 5, **dadurch gekennzeichnet, dass** Drainageröhrchen oder -schläuche am Boden des kegelförmigen Sammelteils (10, 11) befestigt sind.

7. Beutel nach einem der Ansprüche 1 bis 6, **dadurch gekennzeichnet, dass** die gefalteten Teile in den Ausgleichsfalten (12, 13) miteinander in einem oder mehreren Abschnitten (14, 15, 16) entlang der Länge der Ausgleichsfalten verbunden sind.

8. Verfahren zum Herstellen eines Fluidsammelbeutels aus flexiblem Kunststoffmaterial für chirurgische Vorhänge oder Handtücher, der aus zwei Schichten besteht und eine Öffnung, entlang der die Schichten nicht aneinander angebracht sind, und zwei Seitenkanten aufweist, die sich an gegenüberliegenden Seiten der Öffnung erstrecken, entlang derer die Schichten aneinander angebracht sind, **gekennzeichnet durch** die folgenden Schritte:
Zur Verfügung Stellen eines Beutelzuschnitts, der zwei identische, rechteckige Schichten aus flexiblem Kunststoffmaterial mit zwei gegenüberliegenden langen Seiten und zwei gegenüberliegenden kurzen Seiten aufweist, wobei die Schichten so aufeinander angebracht sind, dass ihre langen und kurzen Seiten Kante an Kante liegen, und wobei die Kantenteile entlang einer langen Seite und zumindest einer kurzen Seite miteinander verbunden sind,
danach Ausfalten der langen Seiten voneinander bis sie in derselben Ebene angeordnet sind, wodurch das Ende oder die Enden (10, 11) des Materialstücks, bei dem die Kanten der kurzen Seiten miteinander verbunden sind, eine Kegelform **dadurch** annehmen, dass sie um Faltelinien gefaltet sind, die in der Längenrichtung geneigt sind, und **durch** die kurzen Seitenkanten, die miteinander verbunden sind, die gegen das Materialstück eingefaltet sind und sich entlang der Längensymmetrieachse (A-A) des Beutels erstrecken,
Falten von Ausgleichsfalten der Längskantenteile des Beutelzuschnitts entweder vor oder nach dem Ausfalten der langen Seiten, so dass die Ausgleichsfalten (12, 13) an derselben Seite des Beutelzuschnitts (1) angeordnet sind wie die Kanten der kurzen Seiten, die eingefaltet worden sind, wodurch ein Fluidsammelbeutel gebildet wird.

9. Verfahren nach Anspruch 8, **gekennzeichnet durch** Zur Verfügung Stellen des Beutelzuschnitts **durch** Falten eines Stücks aus flexiblem Kunststoffmaterial (1) mit einer rechteckigen Form, die zwei gegenüberliegende lange Seiten (2, 3) und zwei gegenüberliegende kurze Seiten um eine zentrale Längsfaltelinie (A-A) umfasst, so dass die langen und kurzen Seiten Kante an Kante zueinander angeordnet sind, wonach die zwei gegeneinander gefalteten Endteile zumindest einer der kurzen Seiten miteinander verbunden werden.

10. Verfahren nach Anspruch 8 oder 9, **gekennzeichnet durch** Einfalten des Querkantenteils des kegelförmigen Teils oder der Querkantenteile (17, 18) der kegelförmigen Teile (10, 11) des gebildeten Sammelbeutels über oder unter dem fraglichen Kantenteil nach dem Falten der Ausgleichsfalten der Längskantenteile des Materialstücks.

11. Verfahren nach Anspruch 8, 9 oder 10, **gekennzeichnet durch** Anordnen einer Kopplung (6, 7) an Drainageröhrchen oder -schläuche zwischen den Kantenteilen der kurzen Seiten (4, 5), die gegeneinander eingefaltet sind.

## Revendications

1. Sac collecteur de fluide (23 ; 23' ; 27) fabriqué en matériau synthétique souple pour champs chirurgicaux ou serviettes (24, 25), qui est constitué de deux couches et comprend une ouverture le long de laquelle lesdites couches sont détachées l'une de l'autre, et deux bords latéraux (10A, 10B, 11A, 11B ; 10A', 10B' ; 31, 33 ET 30, 32) s'étendant sur les côtés opposés de l'ouverture le long desquels lesdites couches sont attachées l'une à l'autre, **caractérisé en ce qu'**au moins une partie (12, 13, 10, 11; 12', 13', 10'; 30, 32) d'au moins une des couches du sac comprend au moins un pli de soufflet.

2. Sac selon la revendication 1, **caractérisé en ce que** chacune des parties de bord latérales du sac (12, 13 ; 12', 13' ; 30, 32) comprend au moins une partie contenant un pli de soufflet.

3. Sac selon la revendication 1 ou 2, **caractérisé en ce que** l'une (9 ; 9') des deux couches (9, 10, 11 ; 9', 10') constituant le sac s'étend au-delà du bord de la seconde couche et a des bords latéraux pliés en soufflet (12, 13 ; 12', 13').

4. Sac selon la revendication 3, **caractérisé en ce que** le sac est fabriqué d'une seule pièce de matériau.

5. Sac selon la revendication 4, **caractérisé en ce que** le sac (23) est fabriqué d'une pièce de matériau rectangulaire (1), de sorte que l'une des couches du sac soit constituée de deux parties d'extrémité de forme similaire ayant la forme de triangles rectangles, qui sont repliés l'un contre l'autre et fixés l'un à l'autre dans une couture analogue à un bord (4, 5) et qui, conjointement avec la partie d'extrémité de la pièce de matériau sur laquelle lesdites deux parties d'extrémité de forme similaire ont été pliées, constituent la partie collectrice (10, 11) du sac ayant la forme d'un cône.

6. Sac selon la revendication 5, **caractérisé en ce que** des tuyaux ou tubes de drainage sont fixés à la partie inférieure de la partie collectrice en forme de cône (10, 11).

7. Sac selon l'une quelconque des revendications 1 à 6, **caractérisé en ce que** les parties pliées des plis en soufflet (12, 13) sont raccordées l'une à l'autre en une ou plusieurs parties (14, 15, 16) sur la longueur des plis en soufflet.

8. Procédé de fabrication d'un sac collecteur de fluide fabriqué en matériau synthétique souple pour des champs chirurgicaux ou des serviettes, constitué de deux couches et comprenant une ouverture le long de laquelle lesdites couches ne sont pas attachées l'une à l'autre, et deux bords latéraux s'étendant sur les côtés opposés de l'ouverture le long desquels lesdites couches sont attachées l'une à l'autre, **caractérisé par** les étapes consistant à :
fournir une ébauche de sac comprenant deux couches rectangulaires identiques de matériau synthétique souple ayant deux côtés opposés longs et deux côtés opposés courts, lesdites couches étant placées l'une sur l'autre de sorte que leurs côtés longs et leurs côtés courts se situent bord à bord et ayant les parties de bord le long d'un côté long et au moins un côté court jointes l'une à l'autre ;
déployer ensuite les côtés longs l'un de l'autre jusqu'à ce qu'ils soient dans le même plan, de sorte que la ou les extrémités (10, 11) de la pièce de matériau ayant les bords des côtés courts joints l'un à l'autre adopte(nt) la forme d'un cône par pliage autour de lignes de pliage inclinées dans la direction de la longueur et que les bords des côtés courts liés l'un à l'autre soient repliés contre la pièce de matériau et s'étendent le long de l'axe de symétrie en longueur (A-A) du sac, et
plier en soufflet les parties de bord longitudinales de l'ébauche de sac avant ou après ledit déploiement des longs côtés de sorte que les plis en soufflet (12, 13) se trouvent du même côté de l'ébauche de sac (1) que les bords des côtés courts qui ont été repliés, de manière à former un sac collecteur de fluide.

9. Procédé selon la revendication 8, **caractérisé par** la fourniture de l'ébauche de sac en pliant une pièce de matériau synthétique souple (1) ayant une forme rectangulaire comprenant deux côtés longs opposés (2,3) et deux côtés courts opposés autour d'une ligne de pliage longitudinale centrale (A-A) de sorte que les côtés longs et les côtés courts se situent bord à bord, après quoi les deux parties d'extrémité pliées l'une contre l'autre d'au moins l'un des côtés courts sont jointes l'une à l'autre.

10. Procédé selon la revendication 8 ou 9, **caractérisé par** le pliage de la partie de bord transversale de la partie en forme de cône ou des parties de bord transversales (17, 18) des parties en forme de cône (10, 11) du sac collecteur formé dans, sur ou sous la partie de bord en question après le pliage en soufflet des parties de bord longitudinales de la pièce de matériau.

11. Procédé selon la revendication 8, 9 ou 10, **caractérisé par** le placement d'un couplage (6, 7) avec des tuyaux ou tubes de drainage entre les parties de bord des côtés courts (4,5) repliées l'une contre l'autre.
